Europäisches Patentamt

(19) European Patent Office

Office européen des brevets

(11) Publication number : **0 238 464 B1**

(12) **EUROPEAN PATENT SPECIFICATION**

(45) Date of publication of patent specification :
05.06.91 Bulletin 91/23

(51) Int. Cl.⁵ : **G03B 42/04**

(21) Application number : 87850078.4

(22) Date of filing : 11.03.87

(54) Method and equipment for marking the film in panoramic tomography x-ray apparatuses.

(30) Priority : 21.03.86 FI 861212

(43) Date of publication of application :
23.09.87 Bulletin 87/39

(45) Publication of the grant of the patent :
05.06.91 Bulletin 91/23

(84) Designated Contracting States :
AT BE CH DE ES FR GB GR IT LI LU NL SE

(56) References cited :
DE-A- 2 948 109
DE-A- 3 503 465
DE-B- 2 346 576
US-A- 3 668 394
US-A- 3 683 182

(56) References cited :
US-A- 4 507 797
PATENT ABSTRACTS OF JAPAN, vol. 7, no.
127 (P-201)[1272], 3rd June 1983; & JP - A 58
43448

(73) Proprietor : PLANMECA OY
Mekaanikonkatu 5
SF-00810 Helsinki (FI)

(72) Inventor : Strömmer, Pekka
Ilmakuja 5 F 49
SF-02210 Espoo (FI)
Inventor : Virta, Arto
Pohjolantie 15
SF-01260 Vantaa (FI)

(74) Representative : Onn, Thorsten et al
AB STOCKHOLMS PATENTBYRA Box 3129
S-103 62 Stockholm (SE)

EP 0 238 464 B1

## Description

The present invention relates to a method for marking a film with patient data and possible other information in panoramic tomography X-ray photography, in which method the X-ray beam creating the X-ray photograph and the X-ray film move in a certain way in relation to each other and in which method a certain area of the film is reserved for markings made with the method of the invention, which markings are made latent on the film to become visible when the X-ray film is developed.

The present invention also relates to an apparatus for panomic tomography X-ray photographing equipment for applying the method during X-ray photography for marking patient data or similar information on an X-ray film, preferably on its edge area, simultaneously with the exposure of the X-ray film.

X-ray photographs are usually diagnosed by another person than the person who takes the X-ray photographs. In order to be later able to identify the pictures, the patient's personal data has been recorded on the film after taking the actual photographs ; at the same time also necessary information related to diagnosing, exposure and filing of the pictures has been recorded on the film.

In many countries the law prescribes that the X-ray pictures should be filed ; the X-ray pictures may also be voluntarily filed for follow-up studies and possible medical examinations and care in the future.

In the Prior Art, the X-ray films have generally been marked manually or with a separate marking device. Usually the films are marked manually after development by pasting on the film a stick-on paper label, to which the necessary data is written. A manual marking method is to write the data on a lead-metal tape pasted on the surface of the film cartridge before the X-ray exposure. During the exposure, the text written on the lead tape will be projected on the X-ray film and becomes visible when the film is developed.

The problems with known manual marking are material costs, the required working time, and the possibility of marking a wrong film.

In known separate marking devices the data is written on paper, and the text is projected, by means of a light projector of the device, on an exposed but undeveloped film. The latent marking data on the film will become visible when the film is developed. The marking device is located in the a room, in association with the developing equipment. Several problems have been encountered when using separate film marking devices. The device is used in the dark room which makes it inconvenient to use. The marking strips used in the device are thrown away after one use, which causes material costs. Marking strips are also inconvenient to handle ; there is also a risk that the marking data be confused as the data is not written where the device is located.

As to the State of Art related to the invention, reference is made to US patents Nos. 3668394, 3683182, 3790802, 3845314 and 4121108.

The object of the invention is to provide such a X-ray film marking system, to which additional features can be feasibly added in order to make X-ray film marking more reliable, more versatile, faster and simpler.

In order to attain the aforedescribed objects and other hereinafter set forth, the principal charasteristic feature of the invention is that said markings are made simultaneously with the film exposure to an X-ray beam for X-ray photography, and that said markings are made as a sequence by using, as the deflection parallel with the marking row, the relative movement of the radiation with which the marking is made on the film and the film itself, which movement is synchronized with the relative movement of the X-ray beam, to which the film is exposed for actual X-ray photography, and the film itself.

The first apparatus in accordance with the invention is mainly characterized in that the apparatus comprises a secondary blind arranged between the patient being photographed and the X-ray film, which secondary blind has a set of holes, the direction of the hole set being mainly transverse in relation to the relative movement of the film and the picture-creating X-ray beam, and that in association with said hole set there is a shutter device, with which each hole can be opened and closed under the control of a control/coding unit in such a way that an X-ray beam getting through an opened hole outlines markings on the film according to how it is controlled by the control unit.

The second apparatus in accordance with the invention is mainly characterized in that the apparatus comprises a marking unit which has a set of light sources, which can be turned on and off under the control of the control unit, that in association with the apparatus such a film cartridge is used which has an opening covered with a blind and elongated in the direction of the relative movement of the film and the X-ray beam, that in association with said blind there is a sliding rider arranged to move in said groove of the cartridge, that in the sliding rider there is a set of openings or other parts transparent to radiation, and that said sliding rider can be connected to the part comprising the radiation source set.

In the invention, the film to be marked can be exposed to either light or X-rays. The vertical deflection of the alphanumeric characters of the marking is carried out with superimposed holes passing through the slide. X-rays or light passing through each separate hole can be timed as desired either by opening and closing the hole or by turning on and off a light source located in connection with each hole. The horizontal deflection is provided by linear or rotating movement of the film cartridge.

In the second apparatus in accordance with the invention there is a groove in the edge of the film cartridge, in which groove a perforated rider or slide moves. To the slide movement is synchronized a moving blind in order to prevent the film from being exposed in the marking area except through the holes of the slide. When the cartridge moves, the position of the slide in the cartridge groove changes in relation to the cartridge and the film within the cartridge. The timing of the marking is synchronized with the cartridge movement. The exposure is timed and the control or synchronization of the vertical and horizontal deflection of the characters is controlled or synchrinized preferably by means of a microcomputer. The patient data is entered with a peripheral keyboard.

A device in accordance with the invention preferably comprises a clock which automatically records the date and time information on the film. The X-ray generator provides the exposure parameter data, which is also recorded automatically.

The marking can also be made with X-rays in such a way that a separate perforated blind is arranged into the secondary blind. The vertical deflection is provided by controlled opening and closing of the superimposed holes ; for the horizontal deflection, the movement of the cartridge is made use of. In this kind of system it is possible to use standard-type cartridges.

The invention will now be described in detail with reference to some exemplary embodiments of the invention illustrated in the figures of the accompanying drawing, with not intention to restrict the invention to these details.

Fig. 1 is a schematic illustration of how a panoramic tomography X-ray apparatus for dental photography works.

Fig. 2 schematically illustrates the first embodiment of the invention.

Fig. 3 shows an apparatus in accordance with Fig. 2 seen from direction D and partially as a block diagram presentation.

Fig. 4 illustrates the second embodiment of the invention partially as a block diagram and partially as a cross-section view of the X-ray film cartridge.

Fig. 5 shows an X-ray film cartridge used in a marking system in accordance with Fig. 4 seen from the side of the marking groove.

Fig. 6 shows section VI-VI in Fig. 5.

Fig. 7 shows section VII-VII in Fig. 5.

Fig. 8 illustrates the principle of a character matrix formed in a marking system in accordance with the invention.

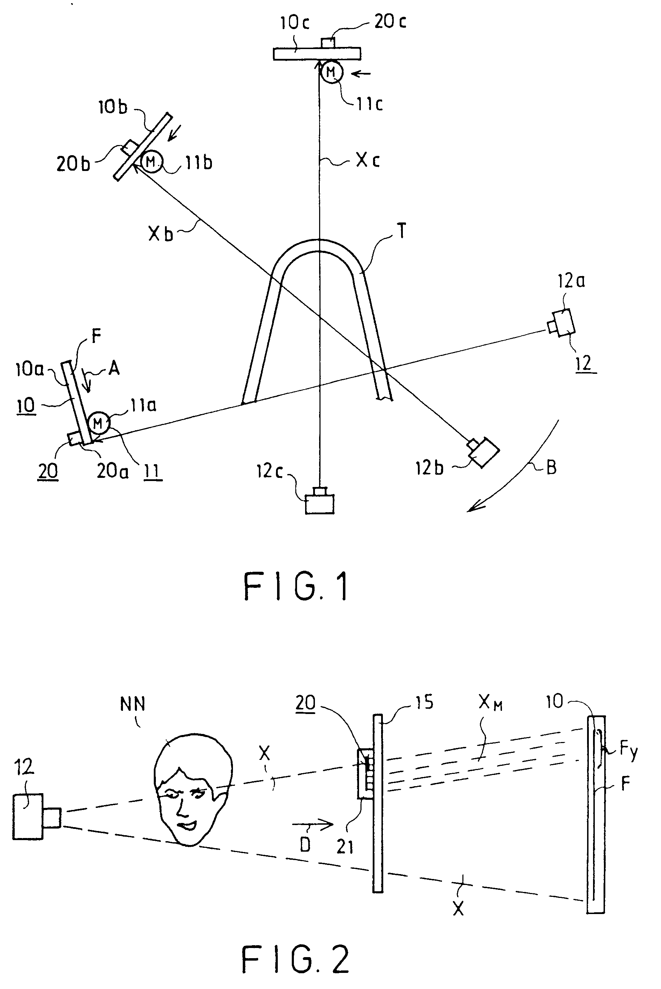

Fig. 1 illustrates the operating principle of a panoramic tomography X-ray apparatus designed for photographing a dental arch T. The X-ray apparatus comprises an X-ray generator 12 and a film cartridge 10, a motor 11 moving the film cartridge in the direction A during exposure, and a film marking device 20 in accordance with this invention. The X-ray generator 12 and the film cartridge 10 with the marking equipment 20 are fastened to each other with a horizontal arm or similar, which is rotated in direction B in such a way that on the film within the cartridge 10 is created a panoramic X-ray photograph representing the dental arch T at the layer to which the device is focussed. As to the moving and controlling mechanisms of the X-ray generator 12 and the film cartridge 10 and as to other details of a panoramic photography X-ray apparatus, reference is made to the Applicant's earlier Finnish Patent Applications No. 852208 (filed June 21, 1985) and No. 853524 (filed September 13, 1985).

The X-ray generator 12a and the film cartridge 10a in association with it, and the marking device 20a in association with the film cartridge 10a begin to move from the position shown in Fig. 1 in direction B, and, through an intermediate position 10b, 11b, 12b, 20b, to the middle position 10c, 11c, 12c, 20c and further to the other end position (not shown). An X-ray beam $X_a$, $X_b$, $X_c$ is aimed from the X-ray-generator 12a, 12b, 12c through the dental arch T, and at the same time the X-ray-generator 12 and the film cartridge 10 rotate around a vertical axis, and the film cartridge and the film F within it move in direction A driven by motor 11. A film marking device 20 in accordance with the invention is fastened to the same frame (not shown) as the motor 11 ; this film marking device 20, specifically during the exposure, records on the film F the patient's personal data and preferably also necessary information related to diagnosing, exposure procedure and/or filing of photographs. A film marking system on accordance with the invention is more closely described below.

Though in the application of Fig. 1, the invention is illustrated in a panoramic tomography X-ray apparatus for dental photography, the invention may as well be applied to other tomogarphy X-ray photography apparatuses, in which there is a film cartridge which moves in relation to the X-ray beam to which the film is exposed.

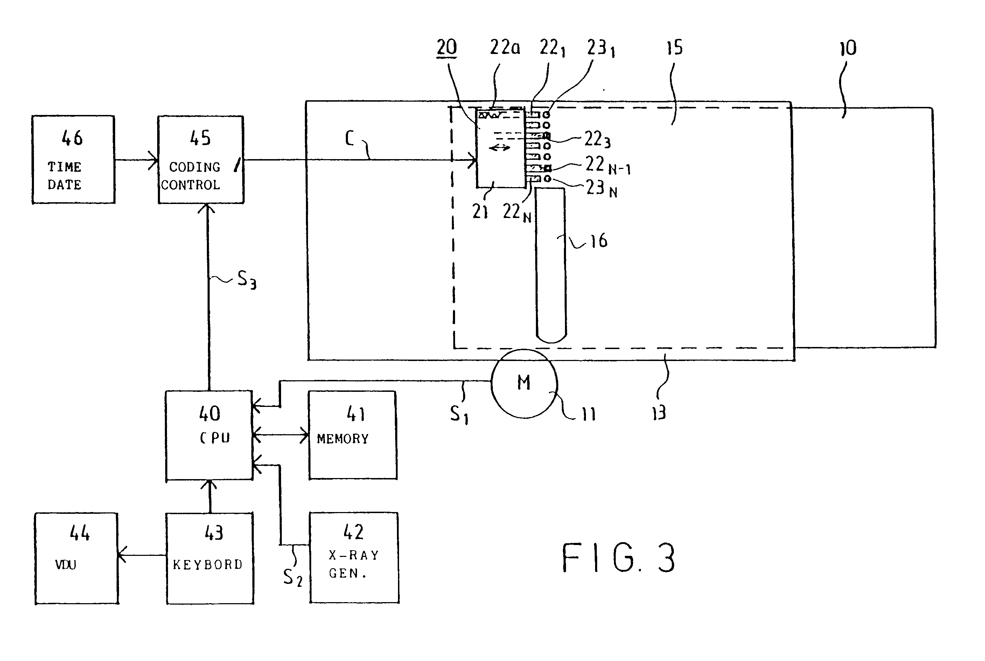

Fig. 2 and 3 illustrate the first embodiment of a film marking apparatus in accordance with the invention. As shown in Fig. 2, the X-ray generator 12 aims an X-ray beam X through the patient NN. Thus a certain layer of for instance the dental arch T of the patient is, in accordance with the principle of Fig. 1, photographed on an X-ray film within the cartridge 10, the top section $F_y$ of the film F being reserved for patient data and other relevant information. Between the film F and the patient NN, in the way of the X-ray beam X, there is a secondary blind 15, which has an opening 16 for the primary radiation beam to enter the film. In the top part of the blind 15 there is a marking device 20 in accordance with the invention, whose design is detailed in Fig. 3.

As shown in Fig. 3, in association with the secondary blind 15 there is a frame section 21 of the marking device 20 having N superimposed slides $22_1$ to $22_N$. In the secondary blind there are, at slides 22, N holes $23_1$ to $23_N$ in a vertical row. In association with the frame section 21 of the marking device 20, each slide 22 has a drive mechanism (not shown), which may comprise for instance solenoids and springs 22a holding the slides 22 in the outer position, in which the slides $22_3$ and $22_{N-1}$ are shown. In an outer position, each slide 22 closes an opening 23 located at it. When a soledoid receives a control current controlled by the coding and control unit 45 (signal c), the slide moves to the opened position thus opening a hole 23 for X-rays X to enter the film F. Because the film is moving, the opened holes 23 outline or "draw" a darker trace on the film F within the cartridge 10.

So the device 20 functions as a marking device, with which the necessary markings can be made under control of the control unit 40-46. In the embodiment in accordance with Fig. 2 and 3, the markings are outlined or "drawn" with X-rays X.

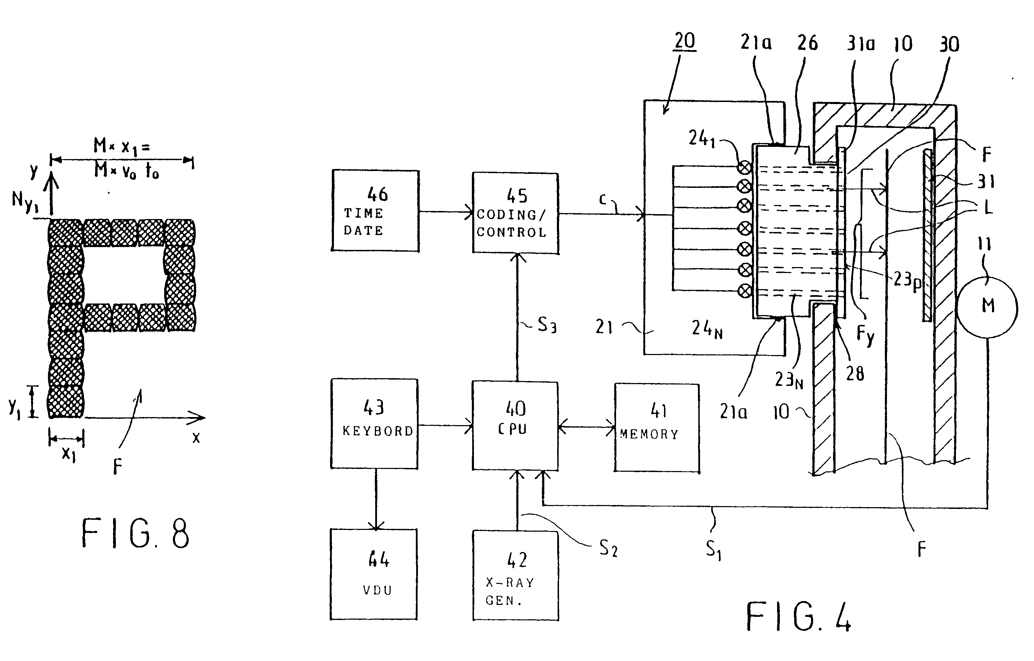

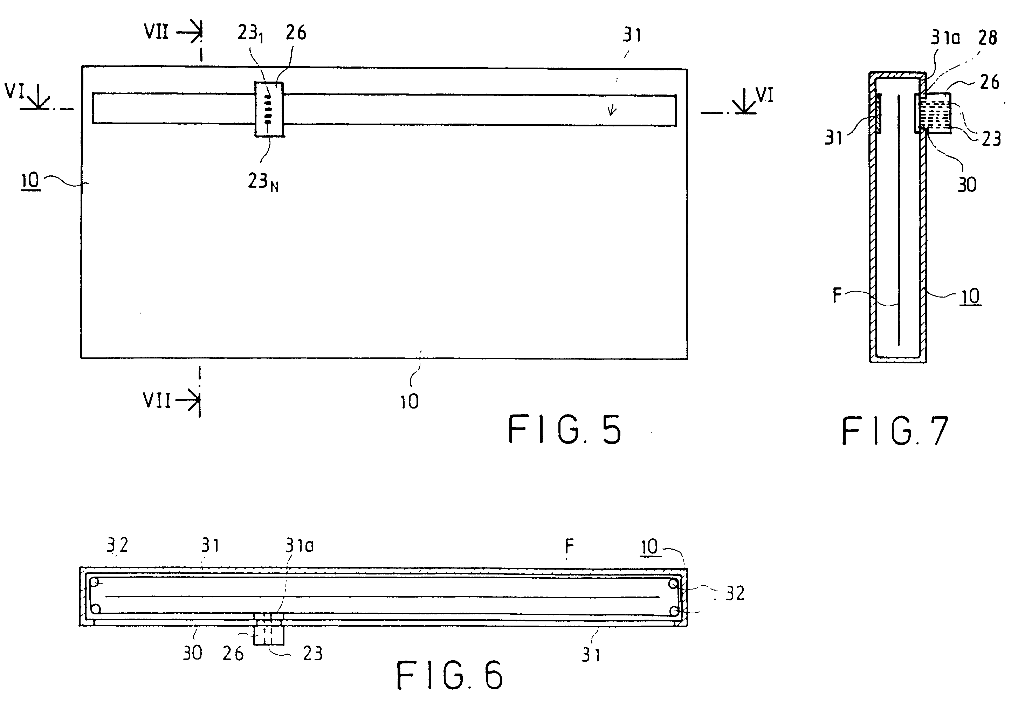

The essential feature of an apparatus in accordance with the invention is that the "horizontal deflection" of the marking is brought about by means of the relative movement between the radiation and the moving X-ray film F, and that the "vertical deflection" is brought about by means of a set of slides 22 and holes 23, or a similar set of holes 23 or optical cables and radiation sources 24, as shown in Fig. 4 to 7. It should be noted that the expressions "vertical deviation" and "horizontal deviation" are used only for clarity, and they are in no way bound to the direction of gravitation.

The control and drive unit of the marking device shown in Fig. 3 comprises a central processing unit (CPU) 40, to which the signal $S_1$ from motor 11 is connected. A memory 41, a keyboard 43 and a video display unit 44 are connected to the central processing unit 40. As a signal $s_2$, the CPU 40 receives from the X-ray generator 42 information about the exposure parameters. In a signal $s_3$, the CPU 40 controls the coding and control unit 45, to which is connected a time and date unit 46. The unit 45 transmits a control signal c to the marking device 20 in accordance with the invention, which, for instance with the matrix principle shown in Fig. 8, outlines on the X-ray film F the personal data typed in with the keyboard 43, the exposure parameter data provided in the signal $s_2$ by the X-ray generator 42, the time and date data provided by the unit 46 and possibly other information for instance stored in the memory 41.

Fig. 4, 5, 6 and 7 illustrate the second embodiment of the invention, in which light is used as film marking radiation. The marking device 20 is installed in association with a groove 30 of the film cartridge 10 particularly designed for this purpose to work as a sort of slide or rider 26, which is hold in position for instance in association with the same frame section (not shown) as the motor 11. In association with the rider 26 there is a blind shaped like a closed loop which covers the opening 30 of the cartridge 10 and prevents the film F being exposed to light through opening 30. The blind 31 is arranged within the cartridge 10 as a closed loop to run around guides 32. When the cartridge 10, driven by motor 11, moves, and the rider 26 of the marking device 20 is fastened to a fixed frame, the rider moves in relation to film F at the same speed as the X-ray beam X moves in relation to film F.

The marking device illustrated in Fig. 4 to 7 comprises a separate part 21, which can with fast couplings be fastened to the rider 26 belonging to the cartridge 10. The rider 26 is fastened so as to move in a groove 27 in association with the groove 30 of the cartridge 10. The rider 26 is fastened to the blind 31 at 31a.

The rider 26 has a set of holes $23_1$ to $23_N$, this set of N holes being positioned crosswise to the moving direction of the rider 26. In a separate part 21 of the marking device 20 there is a set of lamps $24_1$ to $24_N$ located at the outer mouths of the holes 23 of the rider 26, when the part 21 is, with fast-couplings 21a, fastened in association with the rider 26 of the cartridge 10. The inner ends 23p of the holes 23 open near the film F, so that, when turned on, lamps 24 outline with light beams L markings on the top part $F_y$ of the film F, which top part is reserved specifically for this purpose. When required, in association with holes 23 there is an optical system, with which the light beams L are focussed to film F. In the device 20 one can also use optical fibres, so that the light sources 24 themselves may be in the outer part of the rider 26. Therefore the invention can also be so embodied that the unit 20 with the light source 24 is located fixedly outside the rider 26, and between the unit 21 and the rider 26 there is an optical cable, which is connected in association with the rider 26 and its holes 23 with a connector known as such, for instance with a threaded connector corresponding the the fast-Oouupling 21a shown in Fig. 4.

The lamps $24_1$ to $24_N$ are turned on and off with the coding/control unit 45. The marking and control equipment also comprises the units 40-46 described in association with Fig. 3.

In accordance with the invention, the markings are outlined on the film F with X-radiation (Fig. 1 and 2) or with some other radiation, for instance light (Fig. 4 to 7). The markings are preferably made in one row, which is parallel with the relative movement of the X-ray film F and the X-ray beam. The marking is made simultaneously with the photographing exposure. The exposure parameters and other data is automatically received from the central unit 40, and the variable data, for instance the patient's personal data, are typed into the system with the keyboard 43 specifically before the exposure.

Figure 8 shows character P, which is formed in accordance with the invention as a matrix M × N = 5 × 7, and in which the "horizontal deflection" is provided as the relative movement of the radiation source X or L and the film F, and the "vertical deflection" is provided by means of the open/closed or on/off control of the holes of the hole row 23.

In Fig. 8, the size of an element of the matrix is $x_1$ × $y_1$. Thus the size of the character is $Ny_1$ × $Mx_1$. The direction x corresponds with the direction of the relative movement of the X-radiation or light radiation to which the film F is exposed. Therefore, if the velocity of said movement = $v_0$ and the time reserved for an element $x_1$ of the character = $t_0$, the length of the character in the direction of a marking line = $N × V_0 t_0$. The normal space between characters is arranged to be for instance 2 × $x_1$.

The markings can also be made on more than one line, for instance on two or three lines. The whole length of the top edge $F_y$ of the film F or part of it is reserved for markings.

Markings made in accordance with the invention will be latently outlined on the film F by means of radiation X or L, and as the film F is normally developed, the markings will become darker than their surroundings and visible, and will stay visible.

It is charasteristic to the invention that for instance patient data may be typed in with keyboard 43 before the exposure, and other parameters, for instance the exposure parameters, will be moved to the CPU or memory after setting these parameters or during the exposure. In any case, all markings are transferred in an order in accordance with the invention specifically during the exposure, when the film moves in relation to the the X-ray beam. In the invention this is called "horizontal deflection", which, as stated, is in no way bound to the direction of gravitation.

Although the invention has above been described by such an exemplay embodiment in which the film F and its cartridge 10 move linearly in relation to the X-ray beam X to which the film is being exposed, the invention may as well be applied in such known equipment in which the film and its cartridge rotate around a certain axis being simultaneously exposed to an X-ray beam.

The invention is by no means restricted to aforementioned details which are described only as examples ; they may vary within the framework of the inventional idea as set forth in the following claims.

## Claims

1. A method for marking a film with patient data and possible other information in panoramic tomography X-ray photography, in which method the X-ray beam (X) creating the X-ray photograph and the X-ray film (F) move in a certain way in relation to each other and in which method a certain area ($F_y$) of the film (F) is reserved for markings which markings are made latent on the film to become visible when the X-ray film is (F) developed, **characterized in that** said markings are made simultaneously with the film (F) exposure to an X-ray beam (X) for X-ray photography, and that said markings are applied sequentially in a row by using, as the deflection parallel with the marking row, the relative movement of the radiation (X ; L) with which the marking is made on the film and the film (F) itself, which movement is synchronized with the relative movement of the X-ray beam (X), to which the film is exposed for the actual X-ray photography, and the film (F) itself.

2. A method in accordance with claim 1, **characterized in that** the markings are formed with a hole matrix ($23_1$-$23_N$), which is on/off or open/closed controlled, and which provides the control of the deflection transverse (y) to the relative movement (x) of the film (F) and the radiation (X, L) creating an image on the film (F).

3. A method in accordance with claim 1 or 2, **characterized in that** the marking is controlled by a central processing unit (40), preferably a microprocessor, to which the patient's personal data and similar information are entered with a keyboard (43) before the exposure, and that information about the relative movement of the film (F) and the picture-shaping X-ray beam (X) controlling the marking apparatus (20) via the central processing unit (40) and a coding/control unit (45) is also fed to said central processing unit (40).

4. A method in accordance with claim 3, **characterized in that** exposure-parameter information is fed from the X-ray generator (42) to said central processing unit (40) for controlling the central processing unit (40) and the marking apparatus (20), which parameters are, when required, recorded on the film (F) by exposure.

5. A method in accordance with one of claims 1 to 5, **characterized in that** a date/time unit (46) is connected to the said central processing unit (40) and/or coding/control unit (45) for recording date and time information on the film (F) by exposure.

6. A method in accordance with one of claims 1 to 5, **characterized in that** the markings are outlined on the film (F) by using as a drawing (outlining) radiation a part of the X-ray beam (X) creating the X-ray photograph, preferably its edge part ($X_M$), and that between the patient (NN) being photographed and the X-ray film (F) a secondary blind (15) with a hole matrix ($23_1$-$23_N$) is arranged for providing a control transverse to the direction (X) of the relative movement of the X-ray film (F) and the drawing X-ray beam (X) (figures 2 and 3).

7. A method in accordance with one of claims 1 to 5, **characterized in that** a particular electromagnetic radiation (L) is used for outlining the markings, which radiation is guided through the film cartridge (10) through a hole matrix (23, 26) arranged movably in the film cartridge.

8. An apparatus for panoramic tomography X-ray photographing equipment for applying the method in accordance with one of claims 1 to 7 during X-ray photography for marking patient data or similar information on an X-ray film, preferably on its edge area, simultaneously with the exposure of the X-ray film (F), **characterized in that** the apparatus comprises a secondary blind (15) arranged between the patient (NN) being photographed and the X-ray film (F), which secondary blind has a set of holes ($23_1$-$23_N$), the direction of the hole set being mainly transverse in relation to the direction (x) of the relative movement of the film (F) and the picture-creating X-ray beam (X), and that in association with said hole set ($23_1$-$23_N$) there is a shutter device (21, 22, 22a), with which each hole (23) can be opened and closed under the control of a control/coding unit (40-46) in such a way that an X-ray beam getting through an opened hole (23) outlines markings on the film according to how it is controlled (c) by the control unit (40-46).

9. An apparatus in accordance with claim 8, **characterized in that** in the secondary blind (15) there is, at the hole set (23), an opening (16) for the picture-creating X-ray beam (X) (Fig. 3).

10. An apparatus for panomic tomography X-ray photographing equipment for applying the method in accordance with one of claims 1 to 7 during X-ray photography for marking patient data or similar information on an X-ray film, preferably on its edge area, simultaneously with the exposure of the X-ray film (F), **characterized in that** the apparatus comprises a marking unit (20) which has a set of light sources ($24_1$-$24_N$), which can be turned on and off under the control (c) of the control unit (40-46), that the apparatus comprises a film cartridge (10) which has a groove (30) covered with a blind (31) and elongated in the direction (x) of the relative movement of the film (F) and the X-ray beam (X), that in association with said blind (31) there is a sliding rider (26) arranged to move in said groove (30) of the cartridge (10), that in the sliding rider (26) there is a set of openings ($23_1$-$23_N$) or other parts transparent to radiation, and that said rider (26) can be connected to the part (21) comprising the radiation source set ($24_1$-$24_N$).

11. An apparatus in accordance with claim 10, **characterized in that** said blind (31) is arranged in association with said opening (31) of the cartridge (10) as a closed loop being able to move in relation to the cartridge (10) around guides (32) located near the ends of the cartridge (10), and that said blind (31) is arranged to surround the film (F) preferably in its edge area ($F_y$).

12. An apparatus in accordance with one of claims 8 to 11, **characterized in that** the drive and control unit of the apparatus comprises the following interactive components :

the central processing unit (40),
the memory (41),
the keyboard (43),
the date and time unit (46),
the coding and control unit (45) providing the control signal (c) to the marking apparatus (20).

**Ansprüche**

1. Verfahren zum Beschriften eines Films mit Patienten-Daten und gegebenenfalls mit weiteren Informationen in einem Panorama-Röntgen-Tomographen,

bei dem der die Röntgenphotographie erzeugende Röntgenstrahl (X) und der Röntgenfilm (F) eine vorbestimmte Relativbewegung zueinander ausführen

und bei dem ein vorbestimmter Bereich ($F_y$) des Films (F) für Beschriftungen reserviert ist, die auf dem Film latent erzeugt und bei der Entwicklung des Röntgenfilms sichtbar werden, dadurch gekennzeichnet,

daß die Beschriftungen gleichzeitig ausgeführt werden mit der zur Erzeugung der Röntgenphotographie durchgeführten Filmbelichtung durch einen Röntgenstrahl (X)

und daß die Beschriftungen sequentiell in einer Zeile aufgebracht werden, indem für die Ablenkung parallel zu der Beschriftungszeile die Relativbewegung zwischen der Strahlung (X ; L), mit der die Beschriftung ausgeführt wird, und dem Film (F) benutzt wird, wobei diese Relativbewegung mit der Relativbewegung zwischen dem Röntgenstrahl (X), mit der der Film für die tatsächliche Röntgenphotograhie belichtet wird, und dem Film (F) synchronisiert ist.

2. Verfahren nach Anspruch 1, dadurch gekennzeichnet, daß die Beschriftungen mit einer Lochmatrix ($23_1$-$23_N$) ausgeführt werden, die zwischen dem EIN- und dem AUS-Zustand oder zwischen dem offenen oder geschlossenen Zustand umsteuerbar ist und die die Steuerung der Ablenkung quer (y) zu der Relativbewegung (x) zwischen dem Film (F) und der Strahlung (X, L) steuert, die ein Bild auf dem Film (F) erzeugt.

3. Verfahren nach Anspruch 1 oder 2, dadurch gekennzeichnet, daß die Beschriftung durch eine zentrale Prozessoreinheit (40), vorzugsweise einen Mikroprozessor gesteuert wird, in den vor der Belichtung die persönlichen Daten des Patienten und ähnliche Informationen mit Hilfe einer Tastatur (43) eingegeben werden, und daß ferner die Information über die Relativbewegung zwischen dem Film (F) und dem bilderzeugenden Röntgenstrahl (X) zur Steuerung des Beschriftungsgeräts (20) über die zentrale Prozessoreinheit (40) und eine Kodier/Steuereinheit (45) ebenfalls der zentralen Prozessoreinheit (40) zugeführt wird.

4. Verfahren nach Anspruch 3, dadurch gekennzeichnet, daß der zentralen Prozessoreinheit (40) von dem Röntgenstrahl-Generator (42) eine Belichtungsparameter-Information zur Steuerung der zentralen Prozessoreinheit (40) und des Beschriftungsgeräts (20) zugeführt wird, wobei die Belichtungsparameter auf Anforderung auf den Film (F) aufbelichtet werden.

5. Verfahren nach einem der Ansprüche 1 bis 4, dadurch gekennzeichnet, daß mit der zentralen Prozessoreinheit (40) und der Kodier/Steuereinheit (45) eine Datum/Zeit-Einheit (46) zum Aufzeichnen von Datums- und Zeit-Informationen auf dem Film (F) verbunden ist.

6. Verfahren nach einem der Ansprüche 1 bis 5, dadurch gekennzeichnet, daß die Beschriftungen auf dem Film (F) unter Verwendung eines Teils, vorzugsweise des Kantenbereichs (XM), des die Röntgenphotograhie erzeugenden Röntgenstrahls (X) als Aufzeichnungsstrahl ausgeführt werden und daß für die Steuerung quer zu der Richtung (X) der Relativbewegung zwischen dem Röntgenfilm (F) und dem Aufzeichnungs-Röntgenstrahl (X) eine sekundäre Abschirmung (15) mit einer Lochmatrix ($23_1$-$23_N$) zwischen dem zu photographierenden Patienten (NN) und dem Röntgenfilm (F) angeordnet wird (Fig. 2 und 3)

7. Verfahren nach einem der Ansprüche 1 bis 5, dadurch gekennzeichnet, daß zum Aufzeichnen der Beschriftungen eine besondere elektromagnetische Strahlung (L) verwendet wird, die durch eine in der Filmkassette (10) angeordnete Lochmatrix (23, 26) durch die Filmkassette geführt wird.

8. Gerät für eine Panorama-Tomographie-Röntgen-Anlage für die während der Röntgenphotograhie erfolgende Durchführung des Verfahrens nach einem der Ansprüche 1 bis 7 zum Beschriften eines Röntgenfilms, vorzugsweise in seinem Kantenbereich, mit Patienten-Daten oder ähnlichen Informationen gleichzeitig mit der Belichtung des Röntgenfilms (F), dadurch gekennzeichnet,

daß das Gerät eine zwischen dem zu photographierenden Patienten (NN) und dem Röntgenfilm (F) angeordnete sekundäre Abschirmung (15) mit einem Satz von Löchern ($23_1$-$23_N$) aufweist, wobei die Richtung dieses Satzes von Löchern im wesentlichen quer zur Richtung (x) der Relativbewegung zwischen dem Film (F) und dem bilderzeugenden Röntgenstrahl (X) verläuft, und daß in Verbindung mit diesem Satz von Löchern ($23_1$-$23_N$) eine Verschlußvorrichtung (21, 22, 22a) vorgesehen ist, mit der die einzelnen Löcher unter dem Steuereinfluß einer Steuer/Kodiereinheit (40-46) derart geöffnet und geschlossen werden können, daß ein durch ein geöffnetes Loch (23) verlaufender Röntgenstrahl in Abhängigkeit von seiner Steuerung (c) durch die Steuereinheit (40-46) Beschriftungen auf dem Film ausführt.

9. Gerät nach Anspruch 8, dadurch gekennzeichnet, daß in der sekundären Abschirmung (15) im Bereich des Satzes von Löchern (23) eine Öffnung (16) für den bilderzeugenden Röntgenstrahl (X) vorgesehen ist (Fig. 3).

10. Gerät für eine Panorama-Tomographie-Röntgen-Anlage für die während der Röntgenphotograhie erfolgende Durchführung des Verfahrens nach einem der Ansprüche 1 bis 7 zum Beschriften eines Röntgenfilms, vorzugsweise in seinem Kantenbereich, mit Patienten-Daten oder ähnlichen Informationen gleichzeitig mit der Belichtung des Röntgenfilms (F), dadurch gekennzeichnet,

daß das Gerät eine Beschriftungseinheit (20) mit einem Satz von Strahlungsquellen ($24_1$-$24_N$) umfaßt, die unter dem Steuereinfluß (c) der Steuereinheit (40-46) ein- und ausschaltbar sind, daß das Gerät ferner eine Filmkassette (10) umfaßt mit einer von einer Abschirmung (31) abgedeckten Langnut (30), die sich in Richtung (x) der Relativbewegung zwischen dem Film (F) und dem Röntgenstrahl (X) erstreckt, daß in Verbindung mit dieser Abschirmung (31) ein Gleitschieber (26) vorgesehen ist, der in der Nut (30) der Filmkassette (10) bewegbar ist, daß in dem Gleitschieber (26) ein Satz von Öffnungen ($23_1$-$23_N$) oder von anderen strahlungsdurchlässigen Teilen vorgesehen ist, und daß der Gleitschieber (26) mit dem Teil (21) verbindbar ist, das den Satz von Strahlungsquellen ($24_1$-$24_N$) aufweist.

11. Gerät nach Anspruch 10, dadurch gekennzeichnet, daß die Abschirmung (31) in Verbindung mit der Öffnung (31) der Filmkassette (10) als endlose Schleife angeordnet ist, die relativ zu der Filmkassette (10) um Führungen (32) bewegbar ist, die in der Nähe der Enden der Filmkassette (10) liegen, und daß die Abschirmung (31) so angeordnet ist, daß sie den Film (F) vorzugsweise in seinem Kantenbereich ($F_y$) umschließt.

12. Gerät nach einem der Ansprüche 8 bis 10, dadurch gekennzeichnet, daß die Antriebs- und Steuereinheit des Geräts folgende interaktive Komponenten aufweist:
- die zentrale Prozessoreinheit (40),
- den Speicher (41),
- die Tastatur (43),
- die Datums- und Zeit-Einheit (46) und
- die Kodier/Steuereinheit (45) zur Lieferung des Steuersignals (c) an das Beschriftungsgerät (20).

## Revendications

1. Un procédé pour marquer sur un film des données de patient et éventuellement d'autres informations, en tomographie panoramique par rayons X, dans lequel le faisceau de rayons X (X) qui crée la radiographie et le film pour rayons X (F) se déplacent mutuellement d'une certaine manière, et dans lequel une certaine zone ($F_Y$) du film (F) est réservée pour des marquages, ces marquages étant formés de façon latente sur le film pour devenir visibles lorsque le film pour rayons X (F) est développé, caractérisé en ce que ces marquages sont effectués simultanément à l'exposition du film (F) à un faisceau de rayons X (X) pour la radiographie, et en ce que ces marquages sont appliqués séquentiellement en une rangée en utilisant, pour la déflexion parallèlement à la rangée de marquage, le mouvement relatif entre la radiation (X ; L) avec laquelle le marquage est formé sur le film, et le film (F) lui-même, ce mouvement étant synchronisé avec le mouvement relatif entre le faisceau de rayons X (X), auquel le film est exposé pour la radiographie réelle, et le film (F) lui-même.

2. Un procédé selon la revendication 1, caractérisé en ce que les marquages sont formés avec une matrice de trous ($23_1$-$23_N$), qui fait l'objet d'une commande d'activation/désactivation ou d'ouverture/fermeture, et qui assure la commande de la déflexion dans la direction transversale (y) au mouvement relatif (x) entre le film (F) et la radiation (X, L) qui crée une image sur le film (F).

3. Un procédé selon la revendication 1 ou 2, caractérisé en ce que le marquage est commandé par une unité centrale de traitement (40), consistant de préférence en un microprocesseur, dans laquelle les données personnelles du patient et des informations similaires sont introduites au moyen d'un clavier (43) avant l'exposition, et en ce qu'une information concernant le mouvement relatif entre le film (F) et le faisceau de rayons X (X) de formation d'image, qui commande l'appareil de marquage (20) par l'intermédiaire de l'unité centrale de traitement (40) et d'une unité de codage/commande (45), est également fournie à l'unité centrale de traitement (40).

4. Un procédé selon la revendication 3, caractérisé en ce que le générateur de rayons X (42) fournit une information de paramètre d'exposition à l'unité centrale de traitement (40), pour commander cette dernière et l'appareil de marquage (20), et ces paramètres sont enregistrés sur le film (F), par exposition, lorsque c'est nécessaire.

5. Un procédé selon l'une des revendications 1 à 5, caractérisé en ce qu'une unité de date/heure (46) est connectée à l'unité centrale de traitement (40) et/ou à l'unité de codage/commande (45), pour enregistrer une information de date et d'heure sur le film (F), par exposition.

6. Un procédé selon l'une des revendications 1 à 5, caractérisé en ce que les marquages sont dessinés sur le film (F) en utilisant à titre de radiation de dessin une partie du faisceau de rayons X (X) qui crée la radiographie, de préférence une partie marginale ($X_M$) de ce faisceau, et en ce qu'un écran secondaire (15) portant une matrice de trous ($23_1$-$23_N$) est placé entre le patient (NN) qui est radiographié et le film pour rayons X (F), pour réaliser une commande transversalement à la direction (X) du mouvement relatif entre le film pour rayons X (F) et le faisceau de rayons X de dessin (X) (figures 2 et 3).

7. Un procédé selon l'une des revendications 1 à 5, caractérisé en ce qu'on utilise une radiation électromagnétique particulière (L) pour dessiner les marquages, et cette radiation est guidée à travers la cartouche de film (10) par le fait qu'elle traverse une matrice de trous (23, 26) qui est montée de façon mobile dans la cartouche de film.

8. Un appareil pour un équipement de radiographie par rayons X de type tomographique et panoramique, pour la mise en oeuvre du procédé selon l'une des revendications 1 à 7 pendant une opération de radiographie, dans le but de marquer des données de patient ou une information similaire sur un film pour rayons X, de préférence dans une zone marginale du film, simultanément à l'exposition du film pour rayons X (F), caractérisé en ce que l'appareil comprend un écran secondaire (15) qui est placé entre le patient (NN) qui est radiographié et le film pour rayons X (F), cet écran secondaire présentant un ensemble de trous ($23_1$-$23_N$), la direction de l'ensemble de trous étant principalement transversale par rapport à la direction (x) du mouvement relatif entre le film (F) et le faisceau de rayons X de création d'image (X), et en ce qu'un dispositif obturateur (21, 22, 22a) est associé à l'ensemble de trous ($23_1$-$23_N$), chaque trou (23) pouvant être ouvert et fermé sous la commande d'une unité de commande/codage (40-46), d'une manière telle qu'un faisceau de rayons X qui traverse un trou ouvert (23) dessine des marquages sur le film conformément à la manière selon laquelle ce trou est commandé (c) par l'unité de commande (40-46).

9. Un appareil selon la revendication 8, caractérisé en ce qu'une ouverture (16) pour le faisceau de rayons X de création d'image (X) est formée dans l'écran secondaire (15), au niveau de l'ensemble de trous (23) (figure 3).

10. Un appareil pour un équipement de radiographie par rayons X de type tomographique et panoramique, pour la mise en oeuvre du procédé selon l'une des revendications 1 à 7, pendant une opération de radiographie, pour marquer des données de patient ou une information similaire sur un film pour rayons X, de préférence dans une zone marginale du film, simultanément à l'exposition du film pour rayons X (F), caractérisé en ce que l'appareil comprend une unité de marquage (20) qui comporte un ensemble de sources de lumière ($24_1$-$24_N$), qui peuvent être éclairées et éteintes sous la commande (c) de l'unité de commande (40-46), en ce que l'appareil comprend une cartouche de film (10) qui comporte une fente (30) couverte par un rideau (31) et allongée dans la direction (x) du mouvement relatif du film (F) et du faisceau de rayons X (X), en ce qu'un curseur (26) est associé au rideau (31) et est conçu pour se déplacer dans la fente (30) de la cartouche (10), en ce que le curseur (26) contient un ensemble d'ouvertures ($23_1$-$23_N$) ou d'autres éléments transparents au rayonnement, et en ce que le curseur (26) peut être fixé à l'élément (21) qui comprend le jeu de sources de rayonnement ($24_1$-$24_N$).

11. Un appareil selon la revendication 10, caractérisé en ce que le rideau (31) est associé à la fente (30) de la cartouche (10) sous la forme d'une boucle fermée qui peut se déplacer par rapport à la cartouche (10) en passant autour de guides (32) qui sont situés près des extrémités de la cartouche (10), et en ce que le rideau (31) est disposé de façon à entourer le film (F), de préférence dans une zone marginale ($F_Y$) de celui-ci.

12. Un appareil selon l'une des revendications 8 à 11, caractérisé en ce que l'unité de commande de l'appareil comprend les composants interactifs suivants :

l'unité centrale de traitement (40),

la mémoire (41),

le clavier (43),

l'unité de date et d'heure (46),

l'unité de codage et de commande (45) qui applique le siganl de commande (c) à l'appareil de marquage (20).

FIG. 1

FIG. 2

EP 0 238 464 B1

20  22a  22₁  23₁  15  10

46
TIME
DATE

45
CODING
CONTROL

C

22₃
22ₙ₋₁
23ₙ

21  22ₙ

16

S₃

S₁  11  13

M

40
CPU

41
MEMORY

44
VDU

43
KEYBORD

42
X-RAY
GEN.

S₂

FIG. 3

$$M \times x_1 = M \times v_0 \, t_0$$

FIG. 8

FIG. 4

EP 0 238 464 B1

FIG. 5

FIG. 7

FIG. 6

EP 0 238 464 B1